# FASCICULE DE BREVET EUROPEEN

(11) **EP 1 492 583 B1**
(45) Date de publication et mention de la délivrance du brevet: **02.09.2009**
(21) Numéro de dépôt: 02801360.5
(22) Date de dépôt: 04.10.2002
(51) Int. Cl.: A61M 1/02, A61J 1/00, B65D 25/20, G06K 7/10

(54) **PROCEDE DE DETERMINATION ET DE SUIVI DU VIEILLISSEMENT DES POCHES DE SANG**
VERFAHREN ZUR BESTIMMUNG UND ÜBERWACHUNG DER ALTERUNG VON BLUTBEUTELN
METHOD FOR DETERMINING AND MONITORING AGEING OF BLOOD BAGS IN BLOOD TRANSFUSION AND HEALTH CENTRES

(30) Priorité: 18.10.2001 FR 0113410
(43) Date de publication de la demande: 05.01.2005
(73) Titulaire: Biolog S. A., 92517 Boulogne Billancourt (FR)
(72) Inventeur: DE GAULE, Antoine, F-75018 Paris (FR); MONGRENIER, Jean-Claude, F-78100 Saint Germain en Laye (FR)
(74) Mandataire: Schmitt, John
(86) Numéro de dépôt international: PCT/FR2002/003383
(87) Numéro de publication internationale: WO 2003/033052

(56) Documents cités:
- WO-A-00/45331
- FR-A- 2 796 182
- US-A- 6 113 554

## Description

L'invention concerne un procédé de détermination et de suivi du vieillissement des poches de sang dans les établissements de transfusion sanguine et les établissements de soins.

Actuellement les poches de sang sont prélevées dans les établissements de transfusion sanguine ; chaque poche est datée au moment du prélèvement ce qui marque l'origine de la vie de la poche et des produits dérivés pour une durée prédéterminée; les poches de sang prélevées subissent une filtration, une centrifugation et une séparation qui aboutit notamment à un produit dérivé qui est une poche de globules rouges destinée à être transfusée dont la durée de vie prédéterminée est de quarante cinq jours à partir du prélèvement de sang; les poches de globules rouges sont stockées dans les établissements de transfusion sanguine et délivrées au fur et à mesure des besoins des établissements de soins. Il arrive que des poches de globules rouges qui avaient été prévues pour pallier un incident en cours d'opération n'aient pas été utilisées ; comme il est impossible d'avoir actuellement une garantie certaine de la qualité du contenu de la poche de globules rouges, en vue d'une nouvelle utilisation, celle-ci est détruite. La destruction des poches de globules rouges non utilisées représentant, actuellement une perte de douze pour cent, des poches de sang prélevées, qu'il est indispensable de réduire.

Les poches de globules rouges étant actuellement transportées entre l'établissement de transfusion sanguine et l'établissement de soins sans précautions particulières, une étude est en cours avec une société de transport pour garantir la continuité de la chaîne de froid entre ces deux établissements : la poche de sang est ainsi placée dans un conteneur réfrigéré à l'intérieur duquel est placé un dispositif indiquant la température dans le conteneur tout au cours du transport: les poches de globules rouges, étant placées à l'arrivée dans un réfrigérateur approprié de l'établissement de soins, peuvent être retournées à l'établissement de transfusion sanguine si entre temps elles n'ont pas été extraites pour être mises à la disposition d'un chirurgien.

Lors de la prise de sang, le sang est transféré dans une poche de sang mère reliée à un filtre lui-même relié à une poche primaire de sang ; la poche de sang primaire est solidaire d'un groupe de trois poches secondaires auxquelles elle est reliée par des tubulures souples branchées en parallèle; le sang de la poche de sang mère est filtré et introduit dans la poche de sang primaire; la poche de sang primaire est centrifugée pour séparer les globules rouges, des plaquettes et du sérum qui sont ensuite respectivement transférés dans chacune des trois poches et c'est la poche contenant les globules rouges qui est utilisée pour réaliser les transfusions sanguines. Pour mémoire, les plaquettes ne sont isolées que lorsqu'il y a un besoin spécifique à approvisionner ; sinon elles restent avec les globules rouges et il n'y a que deux poches secondaires utilisées.

Un dispositif de traçabilité des poches de sang, selon la demande de brevet FR- 2796182 est en cours de développement ; il associe une puce électronique à la poche de sang; chaque puce électronique comporte une antenne annulaire qui communique avec l'antenne annulaire d'un dispositif de communication électronique, relié à un dispositif informatique, susceptible de fournir à la puce électronique d'une part de l'énergie et d'autre part des informations qu'elle mémorise et qu'elle est susceptible de restituer audit dispositif informatique par l'intermédiaire du dispositif de communication électronique; une puce électronique mère est fixée sur la poche de sang mère qui recueille toutes les informations sur le donneur et les résultats des analyses permettant la qualification de la poche de sang mère; la puce électronique mère est fixée sur un support de puce souple de forme rectangulaire de quelques centimètres de côté sur lequel est imprimé un circuit métallisé en boucles formant l'antenne annulaire de communication; dans une version préférée de l'invention, le support de puce souple de la puce électronique primaire est placé sur une des grandes faces de la poche de sang mère et sous une étiquette rectangulaire recouvrant la plus grande partie d'une face principale; de préférence les supports de puce mère sont toujours placés au même endroit par rapport à l'étiquette de manière à faciliter le positionnement de l'antenne du dispositif de communication électronique. Une demande de brevet FR- 2796182 décrit un peson-agitateur muni d'un dispositif de communication électronique permettant d'enregistrer dans la puce électronique mère de la poche de sang mère les caractéristiques du donneur de sang et les conditions de prélèvement ; le sang de la poche de sang mère est transféré par filtration dans la poche de sang primaire; la poche de sang primaire comporte une puce électronique primaire dans laquelle sont transférées les informations contenues dans la puce électronique mère ainsi que les informations concernant les conditions de filtration; la poche de sang primaire est alors centrifugée; les constituants sont séparés et sont introduits dans les poches secondaires de sang; les poches secondaires de sang sont équipées de puces électroniques secondaires, identiques aux puces électroniques mères et primaires, qui sont fixées sur un support de puce souple placé sous une étiquette recouvrant une face de la poche secondaire de sang et de préférence à une place telle que, lorsque les poches mère, primaire et secondaire de sang sont superposées, les supports de puce électronique mère et primaire ne soient pas superposées entre eux et aux supports de puces souples secondaires; les puces électroniques secondaires, des poches secondaires, c'est-à-dire la poche de globules rouges, la poche de sérum et éventuellement la poche de plaquettes, sont renseignées par le transfert des informations de la puce électronique primaire, de la poche de sang primaire, complétées par les informations concernant les paramètres utilisés pour la séparation des composants sanguins. En ce qui concerne la poche de globules rouges ayant servi à une transfusion sanguine, la puce électronique secondaire qui lui est associée comporte en fin d'utilisation les informations concernant les conditions de son utilisation et notamment l'identité du malade transfusé.

Le procédé, objet de l'invention, est décrit dans la rev. 1 et consiste à formaliser tout au cours de son parcours l'état d'une poche de sang mère, primaire ou secondaire, qu'on appelle ci-après "poche de sang", équipée d'une puce électronique, vis à vis du phénomène de biodégration qu'on qualifie ci-après de "vieillissement", de manière que l'on sache à tout moment de manière la plus précise possible si la poche de sang est qualifiée pour être transfusée.

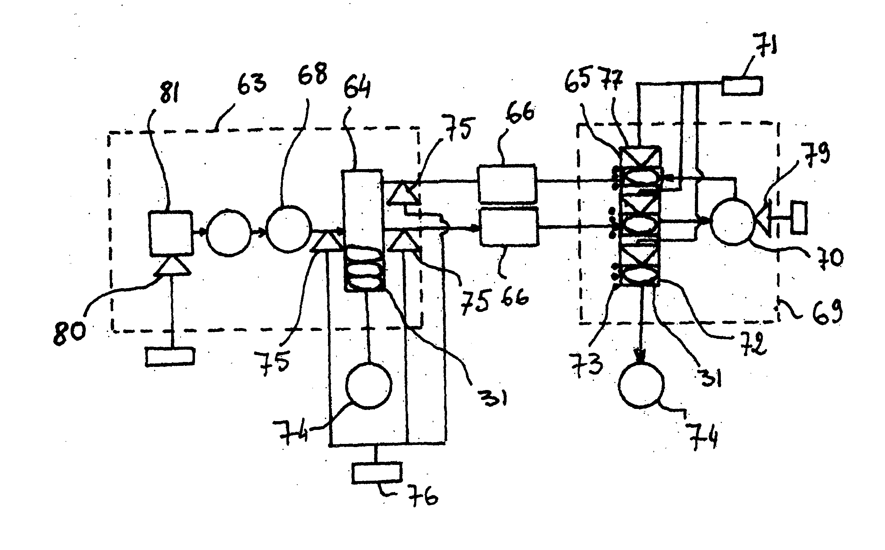

La figure unique représente un schéma de principe des étapes du procédé de qualification, requalification, déqualification d'une poche de sang.

Actuellement, l'établissement de transfusion sanguine 63 délivre une poche de sang qualifiée pendant une période de quarante cinq jours après la date de prélèvement, dans la mesure où elle n'est pas sortie d'une enceinte à atmosphère contrôlée 64 sous le contrôle de l'établissement de transfusion sanguine 63 lui-même; la poche de sang est extraite pour un emploi immédiat dans un établissement de soins 69 et elle est systématiquement détruite si elle n'a pas été utilisée.

L'objet de l'invention consiste à permettre une requalification de la poche de sang qui est sortie de l'enceinte à atmosphère contrôlée 64; pour cela il est alors défini une durée maximum "dT" d'un séjour en dehors d'une enceinte à atmosphère contrôlée 64, 65 : chaque fois que la poche de sang 31 fait un séjour en dehors d'une enceinte à atmosphère contrôlée 64,65, la puce électronique est interrogée grâce à un dispositif de communication électronique 75,77 simplifié équipé d'une antenne en boucle, relié à un automate programmable 76,71 ; si la durée du séjour en dehors de l'enceinte à atmosphère contrôlée est inférieure à dT, la poche de sang est dite "requalifiée" si le délai de quarante-cinq jours depuis le prélèvement n'est pas dépassé ; le délai de quarante-cinq jours permet de définir une date qu'on appelle ci-après "date limite d'utilisation" qui est inscrite dans la puce électronique de la poche de sang mère par un dispositif de communication électronique 80 solidaire d'un peson-agitateur 81 et ensuite transféré aux poches de sang primaires après filtration et secondaires après la phase de séparation 68. Dans ces conditions, lorsque la poche de sang 31 est stockée dans l'enceinte à atmosphère contrôlée 64 de l'établissement de transfusion sanguine 63 après la phase de séparation 68, les seules informations caractérisant son vieillissement est la date limite d'utilisation et la durée maximum de séjour autorisée dT hors d'une enceinte à atmosphère contrôlée 64,65 et la date d'entrée dans l'enceinte à atmosphère contrôlée.

Lorsque la poche de sang 31 est extraite de l'enceinte à atmosphère contrôlée 64 pour être envoyée vers un établissement de soins 69 la date de sortie est comparée à la date limite d'utilisation de la poche de sang 31 et inscrite dans la puce électronique de la poche de sang 31 ; la poche de sang 31 est dite "qualifiée" si la date limite d'utilisation n'est pas dépassée ; si la date limite d'utilisation est dépassée la poche de sang 31 est dite "déqualifiée" et envoyée à la destruction. La poche de sang 31 est transportée vers l'établissement de soins 69 dans un véhicule comportant une enceinte réfrigérée 66 et la poche de sang 31 est requalifiée ou déqualifiée à l'arrivée au moment du transfert de la poche de sang 31 vers l'enceinte à atmosphère contrôlée 65 de l'établissement de soins 69, sur la base de l'enregistrement des températures de l'enceinte réfrigérée 66 en cours de transport ; si la température de transport a été respectée la poche de sang est requalifiée et la date de requalification est inscrite dans la puce électronique de la poche de sang 31 ; si la température de transport n'a pas été respectée pendant une durée inférieure ou égale à dT elle est aussi requalifiée et la date de requalification est inscrite dans la puce électronique de la poche de sang 31; si la durée dT, de non respect de la température de transport, est dépassée la poche est déqualifiée la date n'est pas inscrite et la poche de sang est envoyée à la destruction ; la poche de sang 31 requalifiée est alors introduite dans l'enceinte à atmosphère contrôlée 65 de l'établissement de soins 69.

Lors de l'extraction de la poche de sang 31 de l'enceinte à atmosphère contrôlée 65 pour l'envoyer en salle d'opération 70, la poche de sang 31 est requalifiée ; si la poche de sang 31 n'est pas utilisée et qu'elle est retournée vers l'enceinte à atmosphère contrôlée 65, elle est requalifiée et remise dans l'enceinte à atmosphère contrôlée 65 ou déqualifiée. Dans un souci de fiabilité, il peut être utilisé au niveau de l'établissement de soins une enceinte à atmosphère contrôlée 65 cellulaire du type de celle précédemment décrite dans la demande de brevet FR 0107618 mais qui est gérée par l'automate programmable 71 au lieu d'être gérée par un ordinateur, chaque alvéole 72 étant équipée d'au moins trois lampes témoins 73 ; cet automate programmable 71 vérifie chaque alvéole 72 à intervalles de temps réguliers et rapprochés très inférieurs à dT ; si l'alvéole 72 contient une poche de sang 31, il contrôle la date limite d'utilisation, la date du dernier contrôle inscrit dans la puce électronique et allume celle des trois lampes témoin 73 qui correspond au résultat du contrôle ; il inscrit la nouvelle date de contrôle et allume une lampe témoin verte si la date limite d'utilisation est suffisamment éloignée et le temps écoulé depuis la dernière qualification ou requalification est inférieur à dT, ou il allume une lampe témoin orange si la date limite d'utilisation est proche et le temps écoulé, depuis la dernière requalification, est inférieur à dT ; il allume une lampe témoin rouge sans inscrire dans la puce électronique de la poche de sang la nouvelle date de contrôle pour indiquer que la poche est déqualifiée et doit être détruite dans un incinérateur 74, soit parce que la date limite d'utilisation est dépassée, soit parce que le temps maximum dT est dépassé ; la lampe témoin orange indique aussi, lorsqu'elle est allumée, qu'il faut renvoyer la poche de sang vers l'établissement de transfusion sanguine 63 si l'établissement de soins n'en a pas l'usage immédiat pour éviter la déqualification de la poche de sang, si le délai disponible pour atteindre la date limite d'utilisation est suffisant pour permettre le transfert vers le centre de transfusion sanguine d'une part puis vers un nouvel établissement de soins d'autre part.

Lorsque la poche de sang subit le contrôle ultime par l'intermédiaire d'un dispositif de communication électronique autonome 79 au moment de la transfusion, parmi l'ensemble des paramètres contrôlés, le temps écoulé depuis la sortie de l'atmosphère contrôlée est vérifié et comparé à dT mais ce contrôle n'est pas enregistré dans la puce, comme pour les précédents contrôles, seule la date de transfusion faisant foi.

Si dans le cadre d'un fonctionnement normal la seule détermination d'une durée maximum dT est suffisante pour garantir la qualification d'une poche de sang à être transfusée, il peut être évité des dérives du système en limitant le nombre de séjours ou le temps cumulé des séjours d'une poche de sang en dehors d'une enceinte à atmosphère contrôlée.

## Revendications

1. Procédé de qualification, requalification et déqualification d'une poche de sang, indiquant que cette dernière peut être transfusée ou non sur un patient, sur laquelle est fixée à demeure une puce électronique équipée d'une antenne en boucle susceptible de communiquer avec un dispositif de communication électronique (80) et un dispositif de communication électronique (75,77) simplifié, équipés d'une antenne en boucle, reliés à un automate programmable (76, 71), selon lequel une date limite d'utilisation est définie, à partir d'un instant initial déterminé par un prélèvement dans une poche de sang mère effectué dans un établissement de transfusion sanguine (63) grâce au dispositif de communication électronique (80) solidaire d'un peson-agitateur (81), ladite date limite d'utilisation étant inscrite dans la puce électronique d'une poche de sang mère, puis transférée dans la puce électronique d'une poche de sang primaire, puis dans la puce électronique de poches de sang secondaires et selon lequel est aussi définie une durée maximum de séjour autorisée dT hors d'une enceinte à atmosphère contrôlée (64, 65), permettant de requalifier la poche de sang lors de son retour dans l'enceinte (64, 65) si dT et la date limite d'utilisation ne sont pas dépassées et de la déqualifier si dT ou la date limite d'utilisation sont dépassés afin de la détruire.

2. Procédé selon la revendication 1, **caractérisé en ce que** lorsque la poche de sang est transportée dans un véhicule comportant une enceinte réfrigérée (66) depuis une enceinte à atmosphère contrôlée (64) vers une autre enceinte à atmosphère contrôlée (65), la poche de sang est requalifiée et la date de requalification est inscrite dans la puce électronique si la température de stockage l'enceinte réfrigérée (66) en cours de transport a été respectée ou si la température n'a pas été respectée pendant une durée inférieure ou égale à dT, la poche de sang étant déqualifiée et la date n'étant pas inscrite si la durée dT est dépassée.

3. Procédé selon la revendication 1, **caractérisé en ce que** lorsque la poche de sang (31) est envoyée en salle d'opération (70) elle est requalifiée au moment de son extraction de l'enceinte à atmosphère contrôlée (65), la date de requalification étant alors inscrite dans la puce électronique et si la poche de sang (31) n'est pas utilisée elle est requalifiée, la date de requalification étant inscrite dans la puce électronique et remise dans l'enceinte à atmosphère contrôlée (65) ou déqualifiée.

4. Procédé selon la revendication 1, **caractérisé en ce que** la poche de sang est conservée dans une enceinte à atmosphère contrôlée (65) cellulaire dont chaque alvéole est équipée d'un dispositif de communication électronique spécialisé, l'ensemble étant géré par un automate programmable (71), les poches de sang contenues dans les alvéoles étant contrôlées à intervalle régulier en vue de les requalifier, de les déqualifier ou de détecter si la poche de sang requalifiée approche de la date limite d'utilisation, chaque alvéole étant équipée d'au moins trois lampes témoins (73) qui s'allument en fonction du résultat du contrôle.

5. Procédé suivant la revendication 1, **caractérisé en ce que** la poche de sang (31) subit le contrôle ultime par l'intermédiaire d'un dispositif de communication électronique autonome (79) mais ce contrôle n'est pas enregistré dans la puce électronique.

6. Procédé suivant la revendication 1, **caractérisé en ce que** le nombre de séjours ou le temps cumulé des séjours d'une poche de sang (31) en dehors d'une enceinte à atmosphère contrôlée (64, 65) est limité.

7. Procédé suivant la revendication 1, **caractérisé en ce qu'**une poche de sang est renvoyée vers l'établissement de transfusion sanguine si l'établissement de soins n'en a pas l'utilisation immédiate, si le délai disponible pour atteindre la date limite d'utilisation est suffisant pour permettre le transfert vers le centre de transfusion sanguine d'une part, puis vers un nouvel établissement de soins d'autre part et que le temps écoulé depuis la dernière requalification est inférieur à dT.

## Claims

1. A method for the qualification, requalification and de-qualification of a blood bag indicating whether the latter can be transfused to a patient or not, whereon an electronic chip is permanently fixed and provided with a loop antenna liable to communicate with an electronic communication device (80) and a simplified electronic communication device (75, 77), provided with a loop antenna, connected to a programmable automaton (76, 71), according to which a use-by date is defined, from an initial time determined by a sampling from a parent blood bag, executed in a blood transfusion institution (63) thanks to the electronic communication device (80) integral with a spring type scale-agitator (81), said use-by date being written into the electronic chip of a parent blood bag, then transferred into the electronic chip of a primary blood bag, then into the electronic chip of secondary blood bags and according to which a maximum admissible time dT during which the blood bag can be out of a controlled atmosphere storage enclosure (64, 65) is defined, thus making it possible to requalify the blood bag when the latter is put back into the enclosure (64, 65) if dT and the use-by date are not exceeded and to de-qualify it if dT or the use-by date are exceeded, so that it can be destroyed.

2. A method according to claim 1, **characterized in that**, when the blood bag (31) is transported in a vehicle including a refrigerated enclosure (66) from a controlled atmosphere storage enclosure (64) to another controlled atmosphere storage enclosure (65), the blood bag is re-qualified and the re-qualification date is written into the electronic chip if the storage temperature of the refrigerated enclosure (66) has been complied with during the transportation, or if the temperature has not been complied with for a time less than or equal to dT, the blood bag being de-qualified and the date not being written if the duration dT is exceeded.

3. A method according to claim 1, **characterized in that**, when the blood bag (31) is sent to the operating room (70) it is re-qualified at the moment it is taken out of the controlled atmosphere storage enclosure (65), with the re-qualification date being then written into the electronic chip, and if the blood bag (31) is not used, it is re-qualified, with the requalification date being written into the electronic chip, and being put back into the controlled atmosphere storage enclosure (65) or de-qualified.

4. A method according to claim 1, **characterized in that** the blood bag is kept in a cellular controlled atmosphere storage enclosure (65), each cell of which is provided with a specialised electronic communication device, the assembly being managed by a programmable automaton (71), with the blood bags contained in the cells being controlled at regular intervals so that they can be re-qualified, de-qualified or so as to detect whether the re-qualified blood bag is close to the use-by date, each cell being provided with at least three indicator lights (73) which are lit according to the control result.

5. A method according to claim 1, **characterized in that** the blood bag (31) undergoes the final control through a self-contained electronic communication device (79) but this control is not recorded into the electronic chip.

6. A method according to claim 1, **characterized in that** the cumulative time for which a blood bag (31) is taken out of a controlled atmosphere storage enclosure (64, 65) is limited.

7. A method according to claim 1, **characterized in that** a blood bag is sent back to the blood transfusion institution if the health care facility does not have to use it immediately, if the time available before the use-by date is sufficient for allowing the transfer thereof to the blood transfusion institution, on the one hand and then to a new health care facility on the other hand and if the time elapsed since the last re-qualification is less than dT.

## Patentansprüche

1. Qualifizierungs-, Neuqualifizierungs- und Dequalifizierungsverfahren eines Blutbeutels, das anzeigt, dass dieser auf einen Patienten übertragen werden kann oder nicht, an dem dauerhaft ein elektronischer Chip befestigt ist, der mit einer Spulenantenne ausgerüstet ist, die geeignet ist, mit einer elektronischen Kommunikationsvorrichtung (80) zu kommunizieren, und eine vereinfachte elektronische Vorrichtung (75.77), die beide mit einer Spulenantenne ausgerüstet und mit einem programmierbaren Automaten (76.71) verbunden sind, gemäß dem ein letztes Nutzungsdatum ausgehend von einem Anfangsmoment definiert wird, der durch eine Entnahme in einem Mutterblutbeutel bestimmt wird, welche in einem Bluttransfusionszentrum (63) dank der elektronischen Kommunikationsvorrichtung (80) durchgeführt wird, die fest mit einer Schnellwaage mit Rührwerk (81) verbunden ist, wobei das genannte letzte Nutzungsdatum in den elektronischen Chip eines Mutterblutbeutels eingeschrieben ist, dann in den elektronischen Chip eines primären Blutbeutels übertragen wird, dann in den elektronischen Chip sekundärer Blutbeutel und gemäß dem auch eine maximale zugelassene Verweildauer dT außerhalb einer Einfassung mit kontrollierter Atmosphäre (64,65) definiert wird, die die Neuqualifizierung des Blutbeutels bei seiner Rückkehr in die Einfassung (64.65) erlaubt, wenn dT und das letzte Nutzungsdatum nicht überschritten sind und seine Dequalifizierung, wenn dT oder das letzte Nutzungsdatum überschritten sind, um ihn zu vernichten.

2. Verfahren gemäß Anspruch 1, **dadurch gekennzeichnet, dass**, wenn der Blutbeutel in einem Fahrzeug transportiert wird, das eine gekühlte Einfassung (66) ausgehend von einer Einfassung mit kontrollierter Atmosphäre (64) zu einer anderen Einfassung mit kontrollierter Atmosphäre (65) umfasst, wird der Blutbeutel neu qualifiziert und das Neuqualifizierungsdatum wird in den elektronischen Chip eingeschrieben, wenn die Aufbewahrungstemperatur der gekühlten Einfassung (66) im Verlauf des Transports eingehalten wurde oder wenn die Temperatur während einer Dauer von weniger oder gleich dT nicht eingehalten wurde, wobei der Blutbeutel dequalifiziert wird und das Datum nicht eingeschrieben wird, wenn die Dauer dT überschritten ist.

3. Verfahren gemäß Anspruch 1, **dadurch gekennzeichnet, dass**, wenn der Blutbeutel (31) in den Operationssaal (70) geschickt wird, er zum Zeitpunkt seiner Extraktion aus der Einfassung mit kontrollierter Atmosphäre (65) neu qualifiziert wird, wobei das Neuqualifizierungsdatum dann in den elektronischen Chip eingeschrieben wird und wenn der Blutbeutel (31) nicht verwendet wird, er neu qualifiziert wird, wobei das Neuqualifizierungsdatum in den elektronischen Chip eingeschrieben wird und in der Einfassung mit kontrollierter Atmosphäre (65) übergeben oder dequalifiziert wird.

4. Verfahren gemäß Anspruch 1, **dadurch gekennzeichnet, dass** der Blutbeutel in einer Einfassung mit zellulärer kontrollierter Atmosphäre (65) aufbewahrt wird, von dem jede Zelle mit einer spezialisierten elektronischen Kommunikationsvorrichtung ausgerüstet ist, wobei die Struktur durch einen programmierbaren Automaten (71) verwaltet wird, wobei die in den Zellen enthaltenen Blutbeutel in regelmäßigen Intervallen kontrolliert werden, um sie neu zu qualifizieren, sie zu dequalifizieren oder festzustellen, ob der neu qualifizierte Blutbeutel sich dem letzten Nutzungsdatum nähert, wobei jede Zelle mit wenigstens drei Kontrollleuchten (73) ausgerüstet ist, die in Abhängigkeit von dem Ergebnis der Kontrolle aufleuchten.

5. Verfahren gemäß Anspruch 1, **dadurch gekennzeichnet, dass** der Blutbeutel (31) der letztmaligen Kontrolle über eine autonome elektronische Kommunikationsvorrichtung (79) unterzogen wird, aber diese Kontrolle wird nicht in dem elektronischen Chip eingeschrieben.

6. Verfahren gemäß Anspruch 1, **dadurch gekennzeichnet, dass** die Anzahl von Aufenthalten oder die kumulierte Zeit der Aufenthalte eines Blutbeutels (31) außerhalb einer Einfassung mit kontrollierter Atmosphäre (64.65) begrenzt ist.

7. Verfahren gemäß Anspruch 1, **dadurch gekennzeichnet, dass** ein Blutbeutel zum Bluttransfusionszentrum zurückgeschickt wird, wenn das Pflegeinstitut dafür keine unmittelbare Verwendung hat, wenn die verfügbare Frist bis zum Erreichen des letzten Nutzungsdatums ausreichend ist, um den Transfer einerseits zum Bluttransfusionszentrum, dann zu einem neuen Pflegeinstitut andererseits zu erlauben und wenn die seit der letzten Neuqualifizierung abgelaufene Zeit kürzer ist als dT.
